# EUROPEAN PATENT APPLICATION

(11) **EP 2 602 257 A1**
(43) Date of publication of application: **12.06.2013**
(21) Application number: 11814574.7
(22) Date of filing: 29.07.2011
(51) Int. Cl.: C07D 405/14, C08G 59/26

(54) **EPOXY COMPOUND WITH NITROGEN-CONTAINING RING**

(30) Priority: 05.08.2010 JP 2010176348
(71) Applicant: Nissan Chemical Industries, Ltd., Chiyoda-ku Tokyo 101-0054 (JP)
(72) Inventor: KASAI, Mikio, Tokyo 101-0054 (JP); TAKEYAMA, Toshiaki, Funabashi-shi Chiba 274-8507 (JP); ENDO, Yuki, Funabashi-shi Chiba 274-8507 (JP)
(74) Representative: HOFFMANN EITLE
(86) International application number: PCT/JP2011/067465
(87) International publication number: WO 2012/017948

(57) **Abstract**

There is provided an epoxy compound that provides properties of cured products combining high transparency with high flexural strength by being thermally cured while maintaining advantageous handling properties in a liquid state thereof. An epoxy compound of Formula (1): 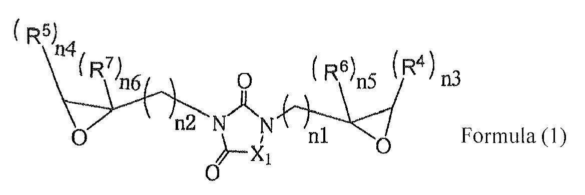 (where n1 and n2 are independently an integer of 2 to 6; n3 and n4 are individually an integer of 2 ; n5 and n6 are individually an integer of 1; R⁴-R⁷ are independently a hydrogen atom or a C₁₋₁₀ alkyl group; and X₁ is a group of Formula (2)-(4): 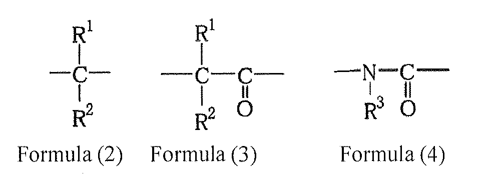 (where R¹-R³ are independently a hydrogen atom, a C₁₋₁₀ alkyl group, or the like)); and a curable composition comprising the epoxy compound and a curing agent.

## Description

### TECHNICAL FIELD

The present invention relates to a thermosetting epoxy compound. Furthermore, the present invention relates to a thermally polymerizable resin composition (a resin composition for electronic materials and optical materials) useful for obtaining a cured product having excellent characteristics such as high adhesion to a substrate, high transparency (transparency for a visible light ray), hard coating property, and high heat resistance, and a production method of a cured product thereof (cured composite).

### BACKGROUND ART

Conventionally, epoxy resins are widely used in the electronic material fields as an epoxy resin composition that combines an epoxy resin and a curing agent.
Among the electronic material fields, for example, in the applications such as a high refractive-index layer in an anti-reflective film (an anti-reflective film for a liquid crystal display and the like), an optical thin film (such as a reflecting plate), a sealant for electronic parts, a printed wiring substrate, and an interlayer insulation film material (such as an interlayer insulation film material for a built-up printed substrate), performances such as high adhesion to a base material, hard coating property, heat resistance, and high transparency for visible light are required for a molding material.
Crystalline epoxy resins generally have a rigid backbone skeleton and are multifunctional, so the crystalline epoxy resins have high heat resistance and are used in a field in which reliability for heat resistance is required such as the electric/electronic field.
In some fields, however, there is an application in which nothing but a liquid composition can be molded, such as in casting molding. Conventional crystalline epoxy resins are limited to be used only in an application in which a solid material is used, such as in transfer molding, and therefore, a range in which the crystalline epoxy resins can be used is limited.
Conventional liquid epoxy resins used for liquid molding, such as casting molding, have such characteristics as advantageous handling and fewer troubles such as an increase in viscosity due to crystallization in the production processes. For this reason, conventional liquid epoxy resins are used for potting, coating, casting, or the like.
These resins are, however, not satisfactory with respect to demand for enhancing properties of cured products, such as heat resistance, for which requirements have become increasingly severe recently in a field such as adhering, casting, sealing, molding, and laminating. Thus, demand for liquefying crystalline multifunctional epoxy resins providing a cured product having high heat resistance has increased. There is also demand for thermally curing the liquid epoxy resins.

As liquid epoxy resins that have been disclosed hitherto, for example, an epoxy resin produced by esterifying a part of epoxy groups of a highly crystalline epoxy compound, for example, tris-(2,3-epoxypropyl)-isocyanurate, to lower crystallinity thereof and liquefying the highly crystalline epoxy compound, is disclosed (see Patent Document 1).
In addition, a compound in which an epoxy ring is bonded to a triazine-trione ring through a long chain alkylene group, is disclosed (see Patent Document 2).
Furthermore, there are disclosed an epoxy compound in which an epoxy ring is bonded to a triazine-trione ring through a long chain alkylene group and an epoxy resin composition using the epoxy compound (see Patent Documents 3, 4, and 5).
In addition, there are disclosed an epoxy compound in which an epoxy cyclohexyl group is bonded to a triazine-trione ring through an oxyalkylene group and an epoxy resin composition using the epoxy compound (Patent Document 6).
An epoxy resin composition containing monoallyl diglycidyl isocyanurate and a curing agent is disclosed (Patent Document 7).

### Related-art Documents

### Patent Documents

Patent Document 1: International Publication No. WO 2006/035641 pamphlet
Patent Document 2: US Patent No. 4376120, specification
Patent Document 3: US Patent Application Publication No. 2007/0295956 specification
Patent Document 4: US Patent Application Publication No. 2007/0295983 specification
Patent Document 5: US Patent Application Publication No. 2007/0299162 specification
Patent Document 6: Japanese Patent Application Publication No. 2010-001424
Patent Document 7: Japanese Patent Application Publication No. 2000-344867

### SUMMARY OF THE INVENTION

### Problems to be Solved by the Invention

In recent years, higher integration of circuits or the use of lead free solder has made the characteristics required for epoxy resin cured products to be used even more severe particularly in the electric/electronic field. Therefore, with conventional liquid epoxy resins, the above characteristics have become difficult to be satisfied.
With an increase in demand for crystalline liquid epoxy resins possessing the characteristics of liquid epoxy resins, such as advantageous handling and fewer troubles in the production thereof such as an increase in viscosity due to crystallization, and providing cured products having excellent physical properties such as high heat resistance that multifunctional epoxy resins have, demand for expanding the application range of epoxy resins have also increased.

The present invention was devised according to the above circumstances and provides: a liquid epoxy resin that is capable of being used as a transparent sealant for an optical semiconductor, for example, a transparent sealant for an LED (light emitting device) and provides properties of cured products combining high transparency with high flexural strength by being thermally cured while maintaining advantageous handling properties in a liquid state thereof; and a composition containing the resin.

### Means for Solving the Problem

As a result of assiduous research intended to overcome these disadvantages, the inventor of the present invention has found that the liquefaction of an epoxy compound containing a nitrogen-containing ring such as hydantoin and cyanuric acid as the main skeleton, and having high heat resistance can be achieved by providing the epoxy compound with a side chain (alkylene group) of a long chain between the nitrogen-containing ring and the epoxy group, and a cured product or a cured coating film capable of compatibilizing excellent mechanical characteristics with excellent optical characteristics can be provided by thermally curing the epoxy compound with a curing agent such as an acid anhydride and an amine, and then, has completed the present invention.

That is, the present invention provides, according to a first aspect, an epoxy compound of Formula (1): 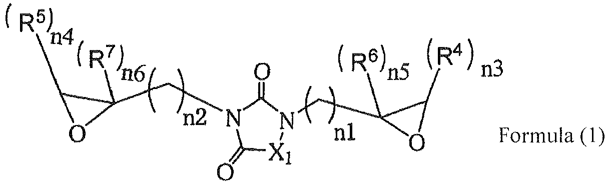 (where n1 and n2 are independently an integer of 2 to 6; n3 and n4 are individually an integer of 2 ; n5 and n6 are individually an integer of 1; R⁴, R⁵, R⁶, and R⁷ are independently a hydrogen atom or a C₁₋₁₀ alkyl group; and X₁ is a group of Formula (2), Formula (3), or Formula (4): 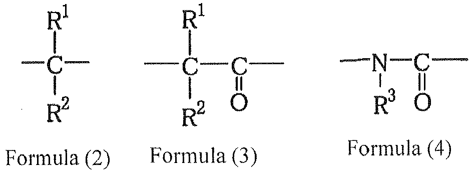 (where R¹, R², and R³ are independently a hydrogen atom, a C₁₋₁₀ alkyl group, a C₂₋₁₀ alkenyl group, a benzyl group, or a phenyl group, where the phenyl group is optionally substituted with a group selected from the group consisting of a C₁₋₁₀ alkyl group, a halogen atom, a C₁₋₁₀ alkoxy group, a nitro group, a cyano group, a hydroxy group, and a C₁₋₆ alkylthio group, and R¹ and R² are optionally bonded with each other to form a C₃₋₆ ring)),
according to a second aspect, the epoxy compound according to the first aspect, in which n1 and n2 are independently an integer of 2 to 4,
according to a third aspect, a curable composition containing the epoxy compound described in the first aspect or in the second aspect and a curing agent,
according to a fourth aspect, the curable composition according to the third aspect, in which the curing agent is an acid anhydride, an amine, a phenolic resin, a polyamide resin, imidazole, or a polymercaptan, and
according to a fifth aspect, the curable composition according to the third aspect or the fourth aspect, in which the curing agent is contained in such a content that the ratio of the curable group of the curing agent reactable with an epoxy group in the epoxy compound relative to the epoxy group is 0.5 to 1.5 equivalents.

### Effect of the Invention

The epoxy compound of the present invention can be obtained as a liquid epoxy compound by achieving the liquefaction of an epoxy compound containing a nitrogen-containing ring such as hydantoin and cyanuric acid as the main skeleton and having high heat resistance. The liquefaction of the epoxy compound is achieved by providing the epoxy compound with a side chain (alkylene group) of a long chain between the nitrogen-containing ring and the epoxy group. Therefore, the epoxy compound of the present invention can be obtained as an epoxy compound excellent in handling properties.
In the epoxy compound of the present invention, the epoxy group bonded to the nitrogen-containing ring through a long chain alkylene group has a large degree of freedom and high reactivity. Therefore, the epoxy compound of the present invention can provide a cured product having high toughness by accelerating the curing reaction of the epoxy compound to enhance the reaction rate of the existing epoxy group, which leads to the stabilization of the glass transition temperature of the obtained cured product, and further, even in a heated environment, the crosslinking density of the cured product is stable and the toughness of the cured product can be maintained. Because the reactivity of the epoxy group is high, the curing reaction is completed in an initial stage of the curing, so that the epoxy compound of the present invention can provide a cured product in which the flexural strength and the elastic modulus are stable even in a heated environment.

The epoxy compound of the present invention is an epoxy compound having a hydantoin skeleton or a cyanuric acid skeleton, so that the epoxy compound has a low viscosity, is excellent in the solubility of a curing agent such as an acid anhydride and an amine, and can easily provide a curable composition thereof by heating-mixing. Furthermore, by having the above skeleton, the epoxy compound of the present invention can provide a cured product having high toughness after the curing thereof.

Because the used epoxy compound has a low viscosity, the curable composition of the present invention is a composition advantageous in filling property and extremely excellent in handling properties.
The curable composition of the present invention has characteristics such as a low viscosity, fast curing, transparency, small shrinkage on curing and can be suitably used for coating or adhering of electronic parts, optical parts, or precision machine parts.

### MODES FOR CARRYING OUT THE INVENTION

### [Epoxy compound]

The target of the present invention is an epoxy compound of Formula (1).
In Formula (1), n1 and n2 are independently an integer of 2 to 6, and preferably, n1 and n2 are independently an integer of 2 to 4. n3 and n4 are individually an integer of 2 and n5 and n6 are individually an integer of 1.
In Formula (1), R⁴, R⁵, R⁶, and R⁷ are independently a hydrogen atom or a C₁₋₁₀ alkyl group.
In Formula (1), X₁ is a group of Formula (2), Formula (3), or Formula (4).

In Formulae (2) to (4), R¹, R², and R³ are independently a hydrogen atom, a C₁₋₁₀ alkyl group, a C₂₋₁₀ alkenyl group, a benzyl group, or a phenyl group, where the phenyl group is optionally substituted with a group selected from the group consisting of a C₁₋₁₀ alkyl group, a halogen atom, a C₁₋₁₀ alkoxy group, a nitro group, a cyano group, a hydroxy group, and a C₁₋₆ alkylthio group, and R¹ and R² are optionally bonded with each other to form a C₃₋₆ ring.

Examples of the C₁₋₁₀ alkyl group include methyl, ethyl, n-propyl, isopropyl, cyclopropyl, n-butyl, isobutyl, sec-butyl, tert-butyl, cyclobutyl, 1-methyl-cyclopropyl, 2-methyl-cyclopropyl, n-pentyl, 1-methyl-n-butyl, 2-methyl-n-butyl, 3-methyl-n-butyl, 1,1-dimethyl-n-propyl, 1,2-dimethyl-n-propyl, 2,2-dimethyl-n-propyl, 1-ethyl-n-propyl, cyclopentyl, 1-methyl-cyclobutyl, 2-methyl-cyclobutyl, 3-methyl-cyclobutyl, 1,2-dimethyl-cyclopropyl, 2,3-dimethyl-cyclopropyl, 1-ethyl-cyclopropyl, 2-ethyl-cyclopropyl, n-hexyl, 1-methyl-n-pentyl, 2-methyl-n-pentyl, 3-methyl-n-pentyl, 4-methyl-n-pentyl, 1,1-dimethyl-n-butyl, 1,2-dimethyl-n-butyl, 1,3-dimethyl-n-butyl, 2,2-dimethyl-n-butyl, 2,3-dimethyl-n-butyl, 3,3-dimethyl-n-butyl, 1-ethyl-n-butyl, 2-ethyl-n-butyl, 1,1,2-trimethyl-n-propyl, 1,2,2-trimethyl-n-propyl, 1-ethyl-1-methyl-n-propyl, 1-ethyl-2-methyl-n-propyl, cyclohexyl, 1-methyl-cyclopentyl, 2-methyl-cyclopentyl, 3-methyl-cyclopentyl, 1-ethyl-cyclobutyl, 2-ethyl-cyclobutyl, 3-ethyl-cyclobutyl, 1,2-dimethyl-cyclobutyl, 1,3-dimethyl-cyclobutyl, 2,2-dimethyl-cyclobutyl, 2,3-dimethyl-cyclobutyl, 2,4-dimethyl-cyclobutyl, 3,3-dimethyl-cyclobutyl, 1-n-propyl-cyclopropyl, 2-n-propyl-cyclopropyl, 1-isopropyl-cyclopropyl, 2-isopropyl-cyclopropyl, 1,2,2-trimethyl-cyclopropyl, 1,2,3-trimethyl-cyclopropyl, 2,2,3-trimethyl-cyclopropyl, 1-ethyl-2-methyl-cyclopropyl, 2-ethyl-1-methyl-cyclopropyl, 2-ethyl-2-methyl-cyclopropyl, and 2-ethyl-3-methyl-cyclopropyl.

Examples of the C₂₋₁₀ alkenyl group include ethenyl, 1-propenyl, 2-propenyl, 1-methyl-1-ethenyl, 1-butenyl, 2-butenyl, 3-butenyl, 2-methyl-1-propenyl, 2-methyl-2-propenyl, 1-ethyl-ethenyl, 1-methyl-1-propenyl, 1-methyl-2-propenyl, 1-pentenyl, 2-pentenyl, 3-pentenyl, 4-pentenyl, 1-n-propyl-ethenyl, 1-methyl-1-butenyl, 1-methyl-2-butenyl, 1-methyl-3-butenyl, 2-ethyl-2-propenyl, 2-methyl-1-butenyl, 2-methyl-2-butenyl, 2-methyl-3-butenyl, 3-methyl-1-butenyl, 3-methyl-2-butenyl, 3-methyl-3-butenyl, 1,1-dimethyl-2-propenyl, 1-isopropyl-ethenyl, 1,2-dimethyl-1-propenyl, 1,2-dimethyl-2-propenyl, 1-cyclopentenyl, 2-cyclopentenyl, 3-cyclopentenyl, 1-hexenyl, 2-hexenyl, 3-hexenyl, 4-hexenyl, 5-hexenyl, 1-methyl-1-pentenyl, 1-methyl-2-pentenyl, 1-methyl-3-pentenyl, 1-methyl-4-pentenyl, 1-n-butyl-ethenyl, 2-methyl-1-pentenyl, 2-methyl-2-pentenyl, 2-methyl-3-pentenyl, 2-methyl-4-pentenyl, 2-n-propyl-2-propenyl, 3-methyl-1-pentenyl, 3-methyl-2-pentenyl, 3-methyl-3-pentenyl, 3-methyl-4-pentenyl, 3-ethyl-3-butenyl, 4-methyl-1-pentenyl, 4-methyl-2-pentenyl, 4-methyl-3-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1-methyl-2-ethyl-2-propenyl, 1-sec-butyl-ethenyl, 1,3-dimethyl-1-butenyl, 1,3-dimethyl-2-butenyl, 1,3-dimethyl-3-butenyl, 1-isobutyl-ethenyl, 2,2-dimethyl-3-butenyl, 2,3-dimethyl-1-butenyl, 2,3-dimethyl-2-butenyl, 2,3-dimethyl-3-butenyl, 2-isopropyl-2-propenyl, 3,3-dimethyl-1-butenyl, 1-ethyl-1-butenyl, 1-ethyl-2-butenyl, 1-ethyl-3-butenyl, 1-n-propyl-1-propenyl, 1-n-propyl-2-propenyl, 2-ethyl-1-butenyl, 2-ethyl-2-butenyl, 2-ethyl-3-butenyl, 1,1,2-trimethyl-2-propenyl, 1-tert-butyl-ethenyl, 1-methyl-1-ethyl-2-propenyl, 1-ethyl-2-methyl-1-propenyl, 1-ethyl-2-methyl-2-propenyl, 1-isopropyl-1-propenyl, 1-isopropyl-2-propenyl, 1-methyl-2-cyclopentenyl, 1-methyl-3-cyclopentenyl, 2-methyl-1-cyclopentenyl, 2-methyl-2-cyclopentenyl, 2-methyl-3-cyclopentenyl, 2-methyl-4-cyclopentenyl, 2-methyl-5-cyclopentenyl, 2-methylene-cyclopentyl, 3-methyl-1-cyclopentenyl, 3-methyl-2-cyclopentenyl, 3-methyl-3-cyclopentenyl, 3-methyl-4-cyclopentenyl, 3-methyl-5-cyclopentenyl, 3-methylene-cyclopentyl, 1-cyclohexenyl, 2-cyclohexenyl, and 3-cyclohexenyl.

Examples of the C₁₋₁₀ alkoxy group include methoxy, ethoxy, n-propoxy, isopropoxy, n-butoxy, isobutoxy, sec-butoxy, tert-butoxy, n-pentoxy, 1-methyl-n-butoxy, 2-methyl-n-butoxy, 3-methyl-n-butoxy, 1,1-dimethyl-n-propoxy, 1,2-dimethyl-n-propoxy, 2,2-dimethyl-n-propoxy, 1-ethyl-n-propoxy, n-hexyloxy, 1-methyl-n-pentyloxy, 2-methyl-n-pentyloxy, 3-methyl-n-pentyloxy, 4-methyl-n-pentyloxy, 1,1-dimethyl-n-butoxy, 1,2-dimethyl-n-butoxy, 1,3-dimethyl-n-butoxy, 2,2-dimethyl-n-butoxy, 2,3-dimethyl-n-butoxy, 3,3-dimethyl-n-butoxy, 1-ethyl-n-butoxy, 2-ethyl-n-butoxy, 1,1,2-trimethyl-n-propoxy, 1,2,2-trimethyl-n-propoxy, 1-ethyl-1-methyl-n-propoxy, and 1-ethyl-2-methyl-n-propoxy.

Examples of the C₁₋₆ alkylthio group include a methylthio group, an ethylthio group, a butylthio group, and a hexylthio group.

Specific examples of the compound of Formula (1) include compounds of Formulae (1-1) to (1-9) below.

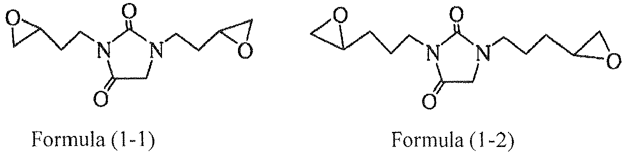

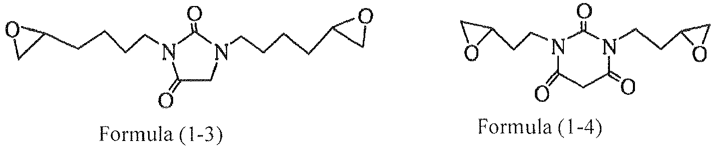

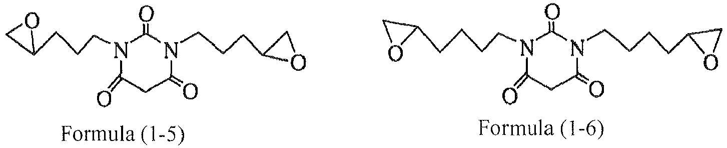

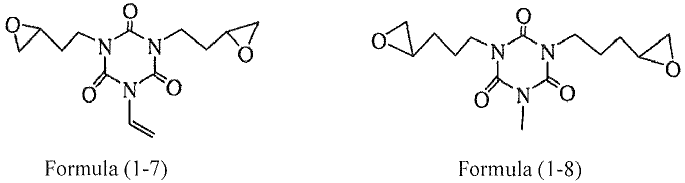

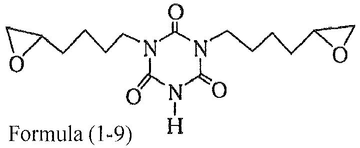

The epoxy compound of Formula (1) of the present invention can be obtained as the objective epoxy compound by a method including: reacting a compound of Formula (5) below and a halogenated alkene (here, halogen is fluorine, chlorine, bromine, or iodine) to generate a compound of Formula (6) as a compound having an unsaturated bond (intermediate); and reacting the compound having an unsaturated bond and a peroxide. 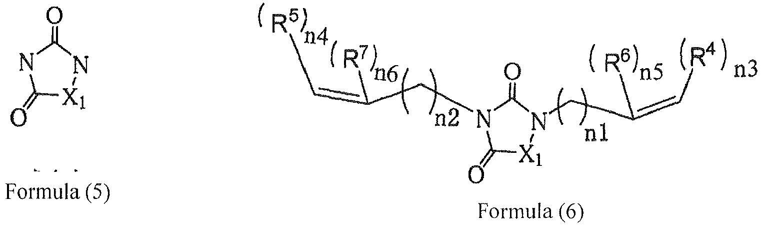 In Formula (5) and Formula (6), X₁ is a group of Formula (2), Formula (3), or Formula (4).
In Formula (6), n1 and n2 are independently an integer of 2 to 6; n3 and n4 are individually an integer of 2; n5 and n6 are individually an integer of 1; and R⁴, R⁵, R⁶, and R⁷ are independently a hydrogen atom or a C₁₋₁₀ alkyl group.

That is, the epoxy compound of the present invention (epoxy compound of Formula (1)) can be obtained as a compound of Formula (1'), for example, using a halogenated alkene of Formula (8), through a compound of Formula (6') as an intermediate, after a reaction of the compound of Formula (6') with a peroxide. 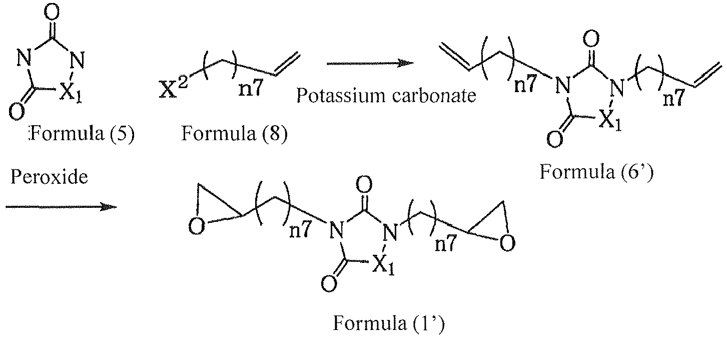 where X₁ is a group of Formula (2), Formula (3), or Formula (4); X₂ is a halogen atom such as a fluorine atom, a chlorine atom, a bromine atom, and an iodine atom; and n7 is n1 or n2.

The reaction of the compound of Formula (5) and a halogenated alkene is effected using a catalyst such as potassium carbonate and using a solvent such as N,N-dimethylformamide at a temperature of 70 to 150°C for 3 to 30 hours.
Then, the peroxide used in the reaction of the obtained compound of Formula (6) having an unsaturated bond and a peroxide is a peroxide containing a peroxy structure or a percarboxylic acid structure and examples thereof include methachloroperbenzoic acid, peracetic acid, and hydrogen peroxide-tungstic acid. This reaction can be effected in a solvent such as dichloromethane and toluene at 0 to 110°C for 1 to 10 hour(s).

### [Curable composition]

The target of the present invention is also a curable composition containing the epoxy compound of Formula (1) and a curing agent.
Examples of the curing agent capable of being used for the curable composition of the present invention include an acid anhydride, an amine, a phenolic resin, a polyamide resin, imidazole, and polymercaptane. Among them, preferred are an acid anhydride and an amine.
The curing agent can be used in such a content that the ratio of the curable group of the curing agent reacted with an epoxy group of the epoxy compound relative to the epoxy group becomes 0.5 to 1.5 equivalents and preferably 0.8 to 1.2 equivalents.
A solid curing agent can be used as a solution prepared by dissolving the solid in a solvent. However, because there is a fear of causing the lowering of the density of the cured product due to evaporation of a solvent and the lowering of the strength and water resistance of the cured product due to formation of a pore after the curing of the curable composition, the curing agent itself is preferably in a liquid state at normal temperature under normal pressure.

### Specific examples of the curing agent are as follows.

As an acid anhydride, an anhydride of a compound having in one molecule thereof, a plurality of carboxy groups is preferred. Specific examples of the acid anhydride include phthalic anhydride, trimellitic anhydride, pyromellitic dianhydride, benzophenonetetracarboxylic anhydride, ethylene glycol bis-trimellitate, glycerol tris-trimellitate, maleic anhydride, tetrahydrophthalic anhydride, methyltetrahydrophthalic anhydride, endo methylenetetrahydrophthalic anhydride, methyl endo methylenetetrahydrophthalic anhydride (called also methyl-5-norbornene-2,3-dicarboxylic anhydride, methylnadic anhydride, or methylhimic anhydride), methylbicyclo [2. 2. 1] heptane-2,3-dicarboxylic anhydride (called also hydrogenated methylnadic anhydride), methylbutenyltetrahydrophthalic anhydride, dodecenylsuccinic anhydride, hexahydrophthalic anhydride, methylhexahydrophthalic anhydride, succinic anhydride, methylcyclohexenedicarboxylic anhydride, and chlorendic anhydride. These acid anhydrides may be used individually or in combination of two or more types thereof.
Among them, preferred are methyltetrahydrophthalic anhydride, methyl endo methylenetetrahydrophthalic anhydride (called also methyl-5-norbornene-2,3-dicarboxylic anhydride, methylnadic anhydride, or methylhimic anhydride), methylbicyclo [2. 2. 1] heptane-2,3-dicarboxylic anhydride (called also hydrogenated methylnadic anhydride), methylbutenyltetrahydrophthalic anhydride, dodecenylsuccinic anhydride, methylhexahydrophthalic anhydride, and a mixture of methylhexahydrophthalic anhydride and hexahydrophthalic anhydride that are in a liquid state at normal temperature under normal pressure. The viscosity of these liquid acid anhydrides measured at 25°C is around 10 mPas to 1,000 mPas.

Examples of the amines include piperidine, N,N-dimethylpiperazine, triethylenediamine, 2,4,6-tris(dimethylaminomethyl)phenol, benzyldimethylamine, 2-(dimethylaminomethyl)phenol, diethylenetriamine, triethylenetetramine, tetraethylenepentamine, diethylaminopropylamine, N-aminoethylpiperazine, di(1-methyl-2-aminocyclohexyl)methane, mencene diamine, isophorone diamine, diaminodicyclohexylmethane, 1,3-diaminomethylcyclohexane, xylenediamine, methaphenylenedimine, diaminodiphenylmethane, and diaminodiphenylsulfon.
Among them, preferred to be used are diethylenetriamine, triethylenetetramine, tetraethylenepentamine, diethylaminopropylamine, N-aminoethylpiperazine, di(1-methyl-2-aminocyclohexyl)methane, mencene diamine, isophoronediamine, and diaminodicyclohexylmethane that are in a liquid state at normal temperature under normal pressure.

Examples of the phenolic resin include a phenol novolac resin and a cresol novolac resin.
Examples of the polyamide resin include a polyamideamine having, in the molecule thereof, a primary amine and a secondary amine that is generated by condensation of a dimer acid and a polyamine.
Examples of the imidazoles include 2-methylimidazole, 2-ethyl-4-methylimidazole, 1-cyanoethyl-2-undecylimidazolium trimellitate, and an epoxy imidazole adduct.
Examples of the polymercaptan include a polymercaptan in which a mercaptan group exists at a terminal of a polypropylene glycol chain and a polymercaptan in which a mercaptan group exists at a terminal of a polyethylene glycol chain, and among them, preferred is a polymercaptan in a liquid state.

When the above cured product is obtained, accordingly, a curing assistant may be used in combination with a curing agent. Examples of the curing assistant include: an organic phosphorus compound such as triphenylphosphine and tributylphosphine; a quaternary phosphonium salt such as ethyltriphenylphosphonium bromide and tetrabutylphosphonium diethylphosphorodithioate; 1,8-diazabicyclo (5, 4, 0) undecane-7-ene; a salt of 1,8-diazabicyclo (5, 4, 0) undecane-7-ene with octylic acid; zinc octylate; and a quaternary ammonium salt such as tetrabutylammonium bromide.
The content of the curing assistant may be 0.001 to 0.1 parts by mass, relative to 1 part by mass of the curing agent.
Alternatively, the curing assistant may be used in a ratio of 0.001 1 to 0.1 equivalents, relative to 1 equivalent of the epoxy group of the epoxy compound.

The curable composition of the present invention may further contain, if necessary, other epoxy compounds, a solvent, a surfactant, an adhesion accelerator, and the like so long as the effect of the present invention is not impaired.

In the present invention, the epoxy compound of Formula (1) can be used in combination with another epoxy compound. The epoxy compound of Formula (1) and the other epoxy compound can be used in a molar ratio of an epoxy group contained in the epoxy compound of Formula (1) : an epoxy group contained in the other epoxy compound = 1:0.1 to 1:0.5.

Examples of the other epoxy compound include compounds of Formulae (9-1) to (9-10) exemplified below.
• Solid epoxy compound: tris-(2,3-epoxypropyl)-isocyanurate (of Formula (9-1), trade name: TEPIC, manufactured by Nissan Chemical Industries, Ltd.) 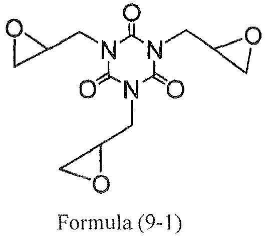
• Liquid epoxy compound: trade name: Epikote 828 (of Formula (9-2), manufactured by Japan Epoxy Resin Co., Ltd. (present: Mitsubishi Chemical Corporation)) 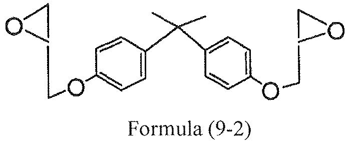
• Liquid epoxy compound: trade name: YX8000 (of Formula (9-3), manufactured by Japan Epoxy Resin Co., Ltd. (present: Mitsubishi Chemical Corporation)) 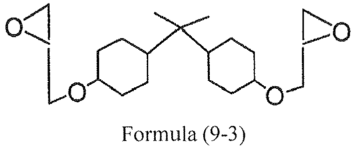
• Liquid epoxy compound: trade name: DME100 (of Formula (9-4), manufactured by New Japan Chemical Co., Ltd.) 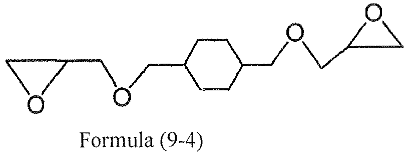
• Liquid epoxy compound: trade name: CEL-2021P (of Formula (9-5), manufactured by Daicel Corporation) 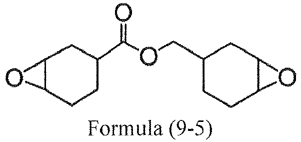
• As liquid epoxy compounds: tris-(3,4-epoxybutyl)-isocyanurate (of Formula (9-6)), tris-(4,5-epoxypentyl)-isocyanurate (of Formula (9-7)), tris-(5,6-epoxyhexyl)-isocyanurate (of Formula (9-8)) 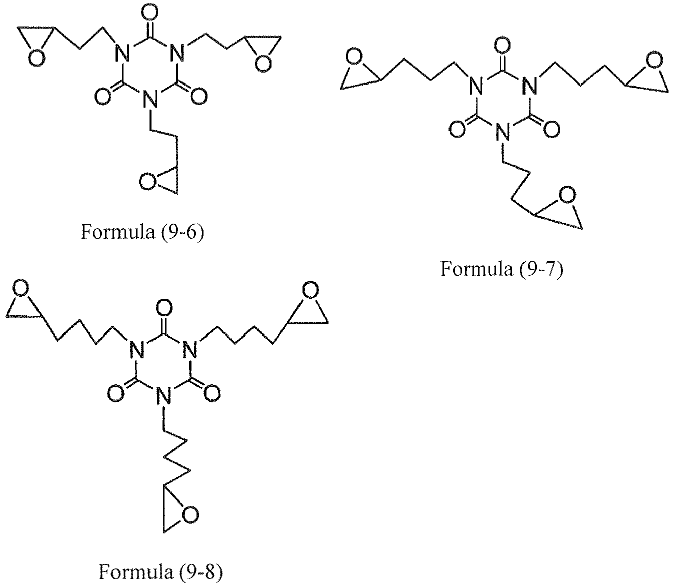
• Liquid epoxy compound: (of Formula (9-9), manufactured by Nissan Chemical Industries, Ltd., trade name: TEPIC-PAS B22) prepared by adding 0.8 mol of propionic anhydride to 1 mol of tris-(2,3-epoxypropyl)-isocyanurate to modify tris-(2,3-epoxypropyl)-isocyanurate. The compound of Formula (9-9) contains a compound of Formula (9-9-1), a compound of Formula (9-9-2), a compound of Formula (9-9-3), and a compound of Formula (9-9-4) in a molar ratio of (9-9-1) : (9-9-2) : (9-9-3) : (9-9-4) = about 35% : 45% : 17% : 3%. 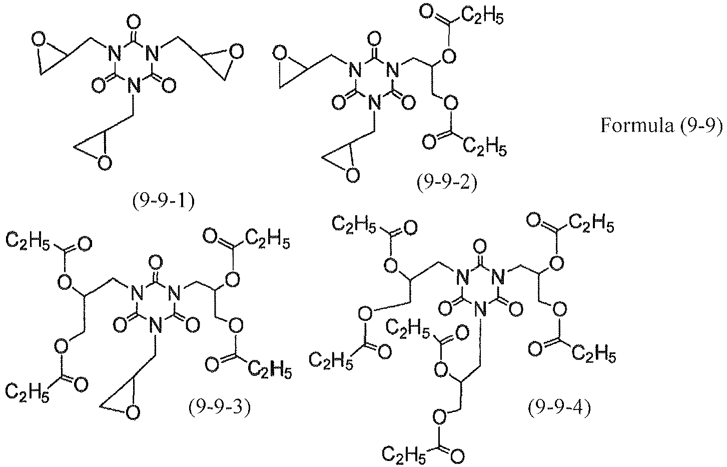
• Liquid epoxy compound: (of Formula (9-10), manufactured by Nissan Chemical Industries, Ltd., trade name: TEPIC-PAS B26) prepared by adding 0.4 mol of propionic anhydride to 1 mol of tris-(2,3-epoxypropyl)-isocyanurate to modify tris-(2,3-epoxypropyl)-isocyanurate. The compound of Formula (9-10) contains a compound of Formula (9-10-1), a compound of Formula (9-10-2), and a compound of Formula (9-10-3) in a molar ratio of (9-10-1): (9-10-2): (9-10-3) = about 60% : 32% : 8%.

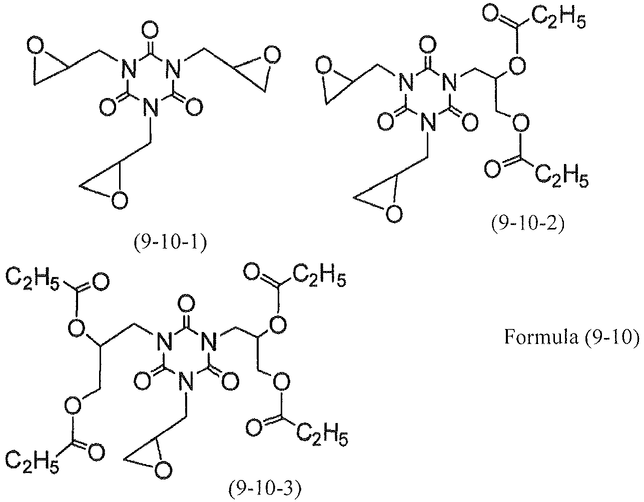

When the curable composition of the present invention contains another epoxy compound, the curing agent can be used in such a content that the ratio of the curable group of the curing agent relative to the total amount of the epoxy group in the epoxy compound of Formula (1) of the present invention and the epoxy group in another epoxy compound becomes 0.5 to 1.5 equivalents and preferably 0.8 to 1.2 equivalents.

In the present invention, the curable composition may contain a solvent as another component. In the present invention, a liquid epoxy compound is used and a curing agent preferably in a liquid state is mixed with the liquid epoxy compound. Therefore, although basically, a solvent is not necessary to be used, it is possible to add a solvent, if necessary.
In the present invention, when the solvent is used, the solid content of the curable composition may be 1 to 100% by mass, or 5 to 100% by mass, or 50 to 100% by mass, or 80 to 100% by mass. The solid content is a content of a component remaining after removing a solvent from the curable composition.

Examples of the solvent include: alcohols such as methanol and ethanol; ethers such as tetrahydrofuran; glycol ethers such as ethylene glycol monomethyl ether and ethylene glycol monoethyl ether; ethylene glycol alkyl ether acetates such as methylcellosolve acetate and ethylcellosolve acetate; diethylene glycols such as diethylene glycol monomethyl ether, diethylene glycol monoethyl ether, diethylene glycol dimethyl ether, diethylene glycol diethyl ether, and diethylene glycol ethyl methyl ether; propylene glycol monoalkyl ethers such as propylene glycol methyl ether, propylene glycol ethyl ether, propylene glycol propyl ether, and propylene glycol butyl ether; propylene glycol alkyl ether acetates such as propylene glycol methyl ether acetate, propylene glycol ethyl ether acetate, propylene glycol propyl ether acetate, and propylene glycol butyl ether acetate; propylene glycol alkyl ether propionates such as propylene glycol methyl ether propionate, propylene glycol ethyl ether propionate, propylene glycol propyl ether propionate, and propylene glycol butyl ether propionate; aromatic hydrocarbons such as toluene and xylene; ketones such as methyl ethyl ketone, cyclohexanone, and 4-hydroxy-4-methyl-2-pentanone; and esters such as methyl acetate, ethyl acetate, propyl acetate, butyl acetate, ethyl 2-hydroxypropionate, methyl 2-hydroxy-2-methylpropionate, ethyl 2-hydroxy-2-methylpropionate, methyl hydroxyacetate, ethyl hydroxyacetate, butyl hydroxyacetate, methyl lactate, ethyl lactate, propyl lactate, butyl lactate, methyl 3-hydroxypropionate, ethyl 3-hydroxypropionate, propyl 3-hydroxypropionate, butyl 3-hydroxypropionate, methyl 2-hydxoxy-3-methylbutanate, methyl methoxyacetate, ethyl methoxyacetate, propyl methoxyacetate, butyl methoxyacetate, methyl ethoxyacetate, ethyl ethoxyacetate, propyl ethoxyacetate, butyl ethoxyacetate, methyl propoxyacetate, ethyl propoxyacetate, propyl propoxyacetate, butyl propoxyacetate, methyl butoxyacetate, ethyl butoxyacetate, propyl butoxyacetate, butyl butoxyacetate, methyl 2-methoxypropionate, ethyl 2-methoxypropionate, propyl 2-methoxypropionate, butyl 2-methoxypropionate, methyl 2-ethoxypropionate, ethyl 2-ethoxypropionate, propyl 2-ethoxypropionate, butyl 2-ethoxypropionate, methyl 2-butoxypropionate, ethyl 2-butoxypropionate, propyl 2-butoxypropionate, butyl 2-butoxypropionate, methyl 3-methoxypropionate, ethyl 3-methoxypropionate, propyl 3-methoxypropionate, butyl 3-methoxypropionate, methyl 3-ethoxypropionate, ethyl 3-ethoxypropionate, propyl 3-ethoxypropionate, butyl 3-ethoxypropionate, methyl 3-propoxypropionate, ethyl 3-propoxypropionate, propyl 3-propoxypropionate, butyl 3-propoxypropionate, methyl 3-butoxypropionate, ethyl 3-butoxypropionate, propyl 3-butoxypropionate, and butyl 3-butoxypropionate.

In the present invention, by mixing the epoxy compound of Formula (1), the curing agent, and, if desired, a curing assistant and other components, a thermosetting composition is obtained. The mixing can be performed using a reaction flask and a stirring blade.
The mixing is performed by a heating-mixing method at a temperature of 10°C to 100°C for 0.5 to 1 hour.

The obtained liquid epoxy resin composition (thermosetting composition) has an appropriate viscosity for being used as a liquid sealant. The liquid thermosetting composition can be prepared to have any viscosity, and for being used as a transparent sealant for an LED or the like by a casting method, a potting method, a dispenser method, a printing method, or the like, the composition can perform partial sealing at any position. By mounting the liquid thermosetting composition in a liquid state as it is by the above method directly on an LED or the like, and then drying and curing the thermosetting composition, an epoxy resin cured product can be obtained.
When the thermosetting composition is used as a sealant, the thermosetting composition is mounted directly on an LED and then, by subjecting the thermosetting composition to preliminary curing at a temperature of 80 to 120°C and to postcuring at a temperature of 120 to 200°C, an epoxy resin cured product can be obtained.
The thermosetting composition is applied to a base material or is poured into a casting plate to which a mold releasing agent is applied, and by subjecting the thermosetting composition to preliminary curing at a temperature of 100 to 120°C and to postcuring at a temperature of 120 to 200°C, a cured product can be obtained.
When the thermosetting composition is applied to a base material, the thickness of the coating film can be selected from a range of around 0. 01 µm to 10 mm depending on the application of the cured product.
As for the heating time, the heating can be performed for 1 to 12 hour(s) and preferably around 2 to 5 hours.

### Example

### (Example 1)

Into a 3-L flask, 75 g of hydantoin, 750 mL of N,N-dimethylformamide, and 332 g of potassium carbonate were charged and while stirring the resultant reaction mixture in a nitrogen atmosphere, 347 g of 5-bromopentene was dropped into the reaction mixture at room temperature. After the completion of the dropping, the reaction mixture was heated at an internal temperature of about 90°C for 24 hours. Then, the reaction vessel was cooled down at room temperature and the content of the reaction vessel was filtered. The resultant filtrate was washed with water three times and then, the filtrate was concentrated to obtain a red black liquid. The liquid was purified with a silica gel column to obtain 144 g of di(4-pentenyl)hydantoin which is an intermediate (vermilion color liquid, yield: 81%).
Next, into a 10-L flask, 144 g of di(4-pentenyl)hydantoin and 6 L of dichloromethane were charged and while cooling down the resultant reaction mixture to 3°C in a nitrogen atmosphere, 784 g of methachloroperbenzoic acid was added to the reaction mixture, followed by stirring the reaction mixture for 7 hours. Then, excessive peroxy acid in the reaction mixture was treated with 2.8 L of a 10% sodium sulfite aqueous solution and then, the resultant reaction mixture was filtered. The filtrate was washed with a sodium bicarbonate aqueous solution and was concentrated to obtain a pale yellow liquid. The liquid was purified with a silica gel column to obtain 133 g of di(4,5-epoxypentyl)hydantoin (compound of Formula (1-2)) (pale yellow liquid, 81%) which is the objective substance.
• H-NMR spectrum values: 1.49 ppm (2H), 1.59-1.83 ppm (6H), 2.49 ppm (2H), 2.77 ppm (2H), 2.94 ppm (2H), 3.40-3.57 ppm (4H), 3.87 ppm (2H)

### (Example 2)

To 24.51g of di(4,5-epoxypentyl)hydantoin obtained in Example 1 (epoxy value = 7.26), 29.2 g of MH-700 (manufactured by New Japan Chemical Co., Ltd., the component thereof is prepared by mixing 4-methylhexahydrophthalic anhydride and hexahydrophthalic anhydride in a molar ratio of 70:30) was added as a curing agent and while heating the resultant reaction mixture in an oil bath of 90°C, the reaction mixture was stirred and degassed for 30 minutes. To the reaction mixture, 245 mg of HISHICOLIN PX-4ET (manufactured by Nippon Chemical Industrial Co., LTD., tetrabutylphosphonium diethylphosphorodithioate) was added and the resultant reaction mixture was stirred and degassed. The reaction mixture was poured in between glass plates (which were treated with a mold releasing agent: SR-2410 (manufactured by Dow Corning Toray Co., Ltd.)) between which a silicone rubber of 3 mm was sandwiched and the reaction mixture was cured by preliminary cure at 100°C for 2 hours and by postcure at 150°C for 5 hours.
The physical properties of the obtained cured product were as follows: flexural strength: 169.4 MPa, flexural modulus: 3369 MPa, deflection until break: 13.59 mm, linear expansion coefficient (30 to 80°C): 65.5 ppm/°C, Tg (TMA): 126.5°C, transmittance (400 nm): 22.0%, boiled water absorption rate (100 h): 4.7%.

### (Comparative Example 1)

To 25.0 g of a liquid epoxy resin (product name: TEPIC-PAS B26 (manufactured by Nissan Chemical Industries, Ltd.), corresponding to a compound of Formula (9-10) (mixture of compounds of Formula (9-10-1) to Formula (9-10-3)), epoxy value = 7.3), 29.86 g of MH-700 (manufactured by New Japan Chemical Co., Ltd.) was added as a curing agent and while heating the resultant reaction mixture in an oil bath of 90°C, the reaction mixture was stirred and degassed for 30 minutes. To the reaction mixture, 252 mg of HISHICOLIN PX-4ET (manufactured by Nippon Chemical Industrial Co., LTD.) was added and the resultant reaction mixture was stirred and degassed. The reaction mixture was poured in between glass plates (which were treated with a mold releasing agent: SR-2410) between which a silicone rubber of 3 mm was sandwiched and the reaction mixture was cured by preliminary cure at 100°C for 2 hours and by postcure at 150°C for 5 hours.
The physical properties of the obtained cured product were as follows: flexural strength: 135.1 MPa, flexural modulus: 3645 MPa, deflection until break: 5.34 mm, linear expansion coefficient (30 to 80°C): 71.8 ppm/°C, Tg (TMA): 182°C, transmittance (400 nm): 90.1%, boiled water absorption rate (100 h): 3.8%.

As shown by the results obtained in Examples, the cured product of the epoxy compound obtained in the present invention (Example 2) exhibited high strength in flexural strength in comparison with a curable composition using liquid epoxy (product name: TEPIC-PAS B26) obtained by modifying triglycidyl isocyanurate (Comparative Example 1).

### INDUSTRIAL APPLICABILITY

The present invention can provide: an epoxy compound having properties of cured products combining high transparency and high flexural strength by thermal curing while maintaining advantageous handling properties in a liquid state; and a curable composition using the compound.
Accordingly, the epoxy compound and the curable composition of the present invention can be used suitably for the adhering of, for example: an optical element such as a lens of a cellular phone or a camera, a light-emitting diode (LED), and a semiconductor laser (LD); parts such as a liquid crystal panel, a biochip, and a lens or a prism of a camera; magnetic parts of a hard disc of a personal computer or the like; a pickup (a part capturing optical information reflected from a disc) of a CD or DVD player; a cone and a coil of a speaker; a magnet of a motor; a circuit substrate; electronic parts; and parts inside an engine of an automobile and the like.
The present invention is applicable to, for example, a body of an automobile or a motorcycle, a lens or a mirror of a head light, a plastic lens of glasses, a cellular phone, a game machine, an optical film, and an ID card as an application to a hard coating material for surface protection of an automobile body, a lamp or electric appliances, a building material, plastic, and the like.
Furthermore, examples of the application of the present invention include applications to cards such as a credit card and a membership card, a printing ink for a switch and a keyboard of electric appliances and OA equipment, and an ink for an inkjet printer for CD, DVD, and the like as an application to an ink material for printing on a metal, such as aluminum, plastic, and the like.
The present invention is applicable also to a technology for producing a complicated three-dimensional object by curing, in combination with a three-dimensional CAD, a resin, a photo fabrication such as modeling of industrial products, coating of an optical fiber, adhering, optical waveguide, thick film resist (for MEMS), and the like.

## Claims

1. An epoxy compound of Formula (1): 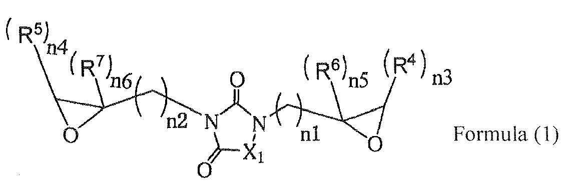 (where n1 and n2 are independently an integer of 2 to 6; n3 and n4 are individually an integer of 2 ; n5 and n6 are individually an integer of 1; R⁴, R⁵, R⁶, and R⁷ are independently a hydrogen atom or a C₁₋₁₀ alkyl group; and X₁ is a group of Formula (2), Formula (3), or Formula (4): 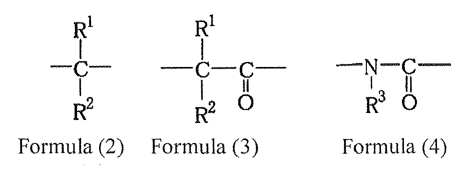 (where R¹, R², and R³ are independently a hydrogen atom, a C₁₋₁₀ alkyl group, a C₂₋₁₀ alkenyl group, a benzyl group, or a phenyl group, where the phenyl group is optionally substituted with a group selected from the group consisting of a C₁₋₁₀ alkyl group, a halogen atom, a C₁₋₁₀ alkoxy group, a nitro group, a cyano group, a hydroxy group, and a C₁₋₆ alkylthio group, and R¹ and R² are optionally bonded with each other to form a C₃₋₆ ring)).

2. The epoxy compound according to claim 1, wherein n1 and n2 are independently an integer of 2 to 4.

3. A curable composition comprising:
the epoxy compound according to claim 1 or 2; and
a curing agent.

4. The curable composition according to claim 3, wherein the curing agent is an acid anhydride, an amine, a phenolic resin, a polyamide resin, imidazole, or a polymercaptan.

5. The curable composition according to claim 3 or 4, wherein the curable composition contains the curing agent in such a content that the ratio of the curable group of the curing agent reactable with an epoxy group in the epoxy compound relative to the epoxy group is 0.5 to 1.5 equivalents.
